# EUROPÄISCHE PATENTSCHRIFT

(11) **EP 3 298 344 B1**
(45) Veröffentlichungstag und Bekanntmachung des Hinweises auf die Patenterteilung: **25.08.2021**
(21) Anmeldenummer: 16729200.2
(22) Anmeldetag: 20.05.2016
(51) Int. Cl.: A61B 1/00, A61B 1/247, A61C 9/00, G01B 11/25

(54) **VERFAHREN UND KAMERA ZUR DREIDIMENSIONALEN VERMESSUNG EINES DENTALEN OBJEKTS**
METHOD AND CAMERA FOR THE THREE-DIMENSIONAL MEASUREMENT OF A DENTAL OBJECT
PROCÉDÉ ET CAMÉRA POUR LA MESURE TRIDIMENSIONNELLE D'UN OBJET DENTAIRE

(30) Priorität: 22.05.2015 DE 102015209404
(43) Veröffentlichungstag der Anmeldung: 28.03.2018
(73) Patentinhaber: Sirona Dental Systems GmbH, 64625 Bensheim (DE)
(72) Erfinder: PFEIFFER, Joachim, 64625 Bensheim (DE); THIEL, Frank, 64372 Ober-Ramstadt (DE)
(74) Vertreter: Özer, Alpdeniz
(86) Internationale Anmeldenummer: PCT/EP2016/061370
(87) Internationale Veröffentlichungsnummer: WO 2016/188881

(56) Entgegenhaltungen:
- EP-A1- 0 300 164
- EP-A1- 2 051 042
- WO-A1-2010/145669
- WO-A1-2012/083967
- US-A1- 2011 287 387

## Beschreibung

### Technisches Gebiet

Die Erfindung betrifft ein Verfahren und eine Kamera zur dreidimensionalen Vermessung eines dentalen Objekts, aufweisend mindestens eine Lichtquelle, die einen Beleuchtungsstrahl abstrahlt, mindestens ein Projektionsmittel, das ein Projektionsmuster erzeugt, eine Fokussierungsoptik, die das Projektionsmuster in einer scharfen Ebene in einem festgelegten Fokusabstand relativ zur dentalen Kamera abbildet, wobei das auf das Objekt projizierte Projektionsmuster als ein Beobachtungsstrahl vom Objekt zurückgestrahlt wird und mittels eines Sensors aufgenommen wird.

### Stand der Technik

Aus dem Stand der Technik sind mehrere Verfahren und Kameras zur dreidimensionalen Vermessung von dentalen Objekten bekannt.

US 2011/0287387 A1 offenbart ein Verfahren zum Abbilden der Oberflächen eines Zahnes, wobei ein Muster auf die Objektoberfläche projiziert wird. Zeitlich versetzt wird eine erste Aufnahme eines ersten Musters und eine zweite Aufnahme eines zweiten Musters aufgenommen. Anschließend wird aus den beiden Aufnahmen ein Restbild erzeugt. Aus dem kombinierten Restbild wird dann ein 3D-Bild der Oberfläche des Zahns berechnet.

In der WO 2012/083967 A1 ist eine Vorrichtung zur optischen 3D-Vermessung eines Objekts unter Verwendung einer optischen konfokalen Messmethode offenbart, wobei zusätzlich zu einer ersten Lichtquelle mindestens eine zweite Lichtquelle verwendet wird, deren Licht unter Verwendung eines Lichtleiters in den Strahlengang der Vorrichtung eingekoppelt wird. Darüber hinaus ist offenbart, dass die Lichtquellen, wie farbliche LED's oder LED's in Kombination mit Farbfiltern, verwendet werden können, wobei die Lichtquellen abwechselnd eingeschaltet werden, um eine homogene Ausleuchtung zu gewährleisten.

Die WO 2010/145669 A1 offenbart eine Vorrichtung zur optischen 3D-Vermessung eines Objekts unter Verwendung einer optischen konfokalen Messmethode. Dabei wird ein sich zeitlich änderndes Muster auf das Objekt projiziert. Das sich ändernde Muster wird mittels eines motorbetriebenen mechanischen Mittels in Form eines Rads erzeugt.

Ein Nachteil dieser Verfahren besteht darin, dass das sich zeitlich ändernde Projektionsmuster unter Verwendung beweglicher Projektionsmittel im Beleuchtungsstrahlengang, wie in Form eines motorbetriebenen, radförmigen Projektionsgitters, erzeugt wird. Durch eine fehlerhafte Ansteuerung bzw. einen fehlerhaften Antrieb der mechanisch angetriebenen Projektionsgitter kann es dabei zu Positionierungsfehlern kommen, die im Ergebnis zu fehlerhaften dreidimensionalen Bilddaten des Objekts führen.

Ein weiterer Nachteil besteht darin, dass die mechanisch angetriebenen Projektionsgitter Bauplatz erfordern und dadurch die Baugröße der Kamera zunimmt.

EP 0 300 164 A1 offenbart ein Verfahren zum optischen 3D Vermessen, wobei das Objekt dreimal nacheinander beleuchtet und aufgenommen wird, wobei zwischen jeder Aufnahme eine Phasenverschiebung der Projektionsstrahlung eines Musters erfolgt.

Die Aufgabe der vorliegenden Erfindung besteht daher darin, eine Kamera bereitzustellen, die kompakt aufgebaut ist und eine fehlerfreie Vermessung des dentalen Objekts ermöglicht.

### Darstellung der Erfindung

Die Erfindung betrifft eine Kamera zur dreidimensionalen Vermessung eines dentalen Objekts, aufweisend mindestens eine Lichtquelle, die einen Beleuchtungsstrahl abstrahlt, mindestens ein Projektionsmittel, das ein Projektionsmuster erzeugt, eine Fokussierungsoptik, die das Projektionsmuster in einer scharfen Ebene in einem festgelegten Fokusabstand relativ zur dentalen Kamera abbildet, wobei das auf das Objekt projizierte Projektionsmuster als ein Beobachtungsstrahl vom Objekt zurückgestrahlt wird und mittels eines Sensors aufgenommen wird. Bei der Vermessung des Objekts wird die Fokussierungsoptik so gesteuert, dass der Fokusabstand der scharfen Ebene relativ zur Kamera schrittweise zwischen mehreren festgelegten Scanpositionen verändert wird, wobei für jede Scanposition mittels des Sensors eine erste Aufnahme und mindestens eine zweite Aufnahme erfolgt. Dabei wird der Sensor seitlich zum Strahlengang des Beobachtungsstrahls oszillierend hin und her bewegt, wobei die erste Aufnahme in einer ersten Sensorposition des Sensors und die zweite Aufnahme in einer zweiten Sensorposition des Sensors aufgenommen wird.

Die Kamera kann in ein herkömmliches Gehäuse in Form eines Handstücks integriert sein. Die Lichtquelle kann beispielsweise eine einzelne LED oder eine Gruppe von LED's sein, die einen Beleuchtungsstrahl mit einem breiten Spektrum abstrahlt. Die Lichtquelle kann also eine weiße LED oder auch eine Kombination von mehreren farblichen LED's sein. Die Lichtquelle kann auch eine Laser-LED bzw. eine Laser-Diode sein, die einen monochromatischen Beleuchtungsstrahl abstrahlt. Das Projektionsmittel kann ein Projektionsgitter bzw. eine Projektionsmaske sein, die das Projektionsmuster erzeugt. Das Projektionsmittel kann auch ein digitaler Lichtprojektor aus Flüssigkristallelementen (LCD) sein, der entsprechend angesteuert wird und das Projektionsmuster erzeugt. Die Fokussierungsoptik ist verstellbar und fokussiert das Projektionsmuster auf die festgelegt scharfe Ebene, wobei die scharfe Ebene schrittweise verändert wird, so dass das Objekt vollständig abgescannt wird. Die Scanpositionen können beispielsweise einen Abstand von 0,1 mm zueinander aufweisen. Durch diesen Abstand wird also die Auflösung entlang einer Scanrichtung festgelegt. Die Verstellung der Fokussierungsoptik kann dabei kontinuierlich erfolgen, wobei lediglich die Bilddaten der Aufnahmen diskret an den festgelegten Scanpositionen ausgelesen werden.

Für jede Scanposition wird die erste Aufnahme und mindestens die zweite Aufnahme erzeugt, wobei der Sensor oszillierend hin und her bewegt wird. Nach diesem Verfahren kann also die Intensität und die Intensitätsänderung für jeden Pixel des Sensors aufgezeichnet werden. Auf diese Weise kann die Differenz der Intensitätswerte in der ersten Aufnahme und in der zweiten Aufnahme bestimmt werden und daraus der Kontrast bzw. die Schärfe der beiden Aufnahmen bestimmt werden. Ausgehend von einem Kontrastwert bzw. einer Schärfe kann dann ein Fokusabstand einer Oberfläche des zu vermessenden Objekts relativ zur scharfen Ebene bestimmt werden. Denn falls die zu vermessende Objektoberfläche von der scharfen Ebene abweicht, erscheint das Objekt in den Aufnahmen unscharf. Bei bekanntem Fokusabstand der scharfen Ebene bzw. der Brennweite der Fokussierungsoptik kann dann der Abstand der Objektoberfläche relativ zur Kamera berechnet werden.

Zur Bestimmung der dreidimensionalen Bilddaten des Objekts kann beispielsweise ein sogenanntes Deph-from-Defocus-Messverfahren (DfD) verwendet werden. Dabei werden die in unterschiedlichen Fokuslagen aufgenommenen Aufnahmen miteinander verrechnet, um dreidimensionale Bilddaten des Objekts zu bestimmen. Dabei wird beispielsweise für jeden Pixel der Intensitätsverlauf als Funktion einer Aufnahmenummer und damit abhängig von der Zeit sowie der Fokuslage aufgetragen. Falls das Objekt sich nicht in der Fokuslage befindet, wird der Kontrast verschlechtert. Falls das Objekt sich in der Fokuslage befindet, wird der Kontrast maximal. Die Aufnahme mit dem maximalen Kontrast wird also in der Fokuslage der Kamera aufgenommen. Auf diese Weise wird die Entfernung des Objekts relativ zu Kamera bestimmt.

Ein Vorteil dieser Kamera besteht darin, dass durch die Bewegung des Sensors im Vergleich zu mechanischen Projektionsmitteln im Beleuchtungsstrahlengang eine kompaktere Bauweise der Kamera ermöglicht wird.

Ein weiterer Vorteil dieser Kamera besteht darin, dass die Verstellung des Sensors zwischen beiden Sensorpositionen sehr präzise angesteuert werden kann, so dass dadurch eine fehlerfreie Vermessung des Objekts ermöglicht wird.

Vorteilhafterweise kann das Projektionsmuster ein schachbrettförmiges Muster sein und aus dunklen und hellen quadratischen Musterelementen bestehen.

Durch das schachbrettförmige Muster wird auf eine einfache Art und Weise durch die Verschiebung des Sensors um ein Musterelement ein schneller Wechsel zwischen dunklen und hellen Musterelementen ermöglicht.

Vorteilhafterweise kann das Projektionsmittel so bemessen und ausgerichtet sein, dass jedes Musterelement des Projektionsmusters auf einen Pixel des Sensors projiziert wird, so dass das projizierte Bild des Musterelements in der Ebene des Sensors den Abmessungen des Pixels entspricht.

Dadurch wird ein einzelnes Musterelement auf einen einzelnen Pixel projiziert, so dass bei der oszillierenden Bewegung des Sensors abwechselnd ein dunkles und ein helles Musterelement auf jedes Pixel projiziert wird. Dadurch wird also auf einfache Art und Weise ermöglicht für jedes Pixel die Differenz zwischen beiden Intensitätswerten in der ersten Aufnahme und in der zweiten Aufnahme zu bestimmen. Falls die Oberfläche des aufzunehmenden Objekts in der scharfen Ebene liegt, wird dieses Muster scharf abgebildet, so dass die Differenz der Intensitätswerte und damit der Kontrast im Maximum liegt. Auf diese Weise lassen sich die einzelnen Punkte des Objekts vermessen und daraus die dreidimensionalen Bilddaten des Objekts erzeugen.

Vorteilhafterweise kann der Sensor bei der oszillierenden Bewegung um einen Abstand zwischen der ersten Sensorposition und der zweiten Sensorposition versetzt werden, der der Breite eines Pixels des Sensors entspricht.

Dadurch ist die Bewegung des Sensors zwischen der ersten Sensorposition und der zweiten Sensorposition minimal klein und kann beispielsweise mittels eines Piezoelements bewerkstelligt werden. Alternativ kann der Abstand der seitlichen Bewegung auch einem Vielfachen der Breite des Pixels entsprechen.

Vorteilhafterweise kann der Sensor entlang einer ersten Sensorachse parallel zu den Zeilen oder entlang einer zweiten Sensorachse parallel zu den Spalten bewegt werden.

Dadurch kann die Verstellung des Sensors auf eine einfache Art und Weise entlang der Zeilen oder entlang der Spalten erfolgen.

Vorteilhafterweise kann die Kamera eine Beobachtungsmaske im Strahlengang des Beobachtungsstrahls vor dem Sensor aufweisen, die zusammen mit dem Sensor zwischen den beiden Sensorpositionen mitbewegt wird.

Die Beobachtungsmaske kann beispielsweise wie das Projektionsmuster ebenfalls einer schachbrettförmigen Struktur aufweisen.

Vorteilhafterweise kann die Beobachtungsmaske ein Bayer-Filter sein, der eine schachbrettförmige Struktur aus roten, grünen und blauen Farbfiltern umfasst, die jeweils einem Pixel des Sensors zugeordnet sind, so dass eine Farbmessung des dentalen Objekts ermöglicht wird.

Dadurch wird also jedem Pixel ein bestimmter Farbfilter vorgeschaltet, so dass beim Auswerten der einzelnen Intensitätswerte der Pixel die Farbe des Objekts bestimmt werden kann. Dadurch können also die dreidimensionale Vermessung und die Farbmessung gleichzeitig bei einem gemeinsamen Tiefenscan erfolgen.

Die verschiedenen Farbfilter können auch andere Farben aufweisen, die geeignet sind eine Farbmessung durchzuführen. Ein Farbfilter kann auch eine Gruppe von Pixeln des Sensors, wie einer 2 x 2 oder einer 4 x 4 Gruppe von Pixeln, zugeordnet sein.

Vorteilhafterweise kann unter Verwendung der ersten Aufnahme in der ersten Sensorposition und der zweiten Aufnahme in der zweiten Sensorposition, die um einen Pixel relativ zur erstes Sensorposition versetzt ist, für jeden Pixel des Sensors ein erster Intensitätswert in der ersten Sensorposition und ein zweiter Intensitätswert in der zweiten Sensorposition ermittelbar sein, wobei mittels einer Recheneinheit der Kamera durch Differenzbildung ein Differenzwert zwischen dem ersten Intensitätswert und dem zweiten Intensitätswert ermittelbar sein kann.

Dadurch wird also der Differenzwert zwischen den beiden Intensitätswerden ermittelt, der einem Kontrastwert entspricht. Dadurch kann also die Änderung des Differenzwertes beim Verstellen zwischen den einzelnen Scanpositionen ermittelt werden, wobei das Maximum dieses Differenzwertes der Tiefenposition der Oberfläche des Objekts entspricht.

Vorteilhafterweise kann unter Verwendung des Differenzwertes in Abhängigkeit vom Fokusabstand für jeden Pixel eine Tiefeninformation einer Objektoberfläche des Objekts mittels der Recheneinheit ermittelt werden, so dass auf diese Weise dreidimensionale Oberflächendaten des Objekts erzeugt werden.

Dadurch wird also für mehrere Messpunkte der Objektoberfläche die Tiefeninformation ermittelt, so dass daraus die dreidimensionalen Oberflächendaten des Objekts erzeugt werden können.

Vorteilhafterweise kann das Projektionsmuster aus mehreren parallelen hellen und dunklen Streifen bestehen.

Dadurch wird also ein herkömmliches Projektionsmuster aus parallelen Streifen verwendet.

Vorteilhafterweise kann das Projektionsmittel so bemessen und ausgerichtet sein, dass jeder Streifen des Projektionsmusters auf eine Spalte oder eine Zeile von Pixeln des Sensors projiziert wird, so dass die Breite eines projizierten Streifens in der Ebene des Sensors der Breite des Pixels entspricht.

Dadurch wird also jeder Streifen des Projektionsmusters auf eine Spalte bzw. eine Zeile des Sensors projiziert.

Vorteilhafterweise kann der Sensor bei der oszillierenden Bewegung um einen Abstand zwischen der ersten Sensorposition und der zweiten Sensorposition versetzt werden, der der Breite eines Pixels des Sensors entspricht, wobei der Sensor senkrecht zu den projizierten Streifen bewegt wird.

Dadurch wird also durch die oszillierende Bewegung des Sensors der Sensor lediglich um die Breite eines Pixels versetzt, wobei auf jedes Pixel abwechselnd ein heller Streifen oder ein dunkler Streifen des Projektionsmusters projiziert wird.

Vorteilhafterweise kann die Kamera bei einem Projektionsmuster aus parallelen Streifen eine Beobachtungsmaske im Strahlengang des Beobachtungsstrahls vor dem Sensor aufweisen, die mit dem Sensor mitbewegt wird, wobei die Beobachtungsmaske ein Bayer-Filter ist, der aus einer schachbrettförmigen Struktur aus roten, grünen und blauen Farbfiltern besteht, die jeweils einem Pixel des Sensors zugeordnet sind, so dass eine Farbmessung des dentalen Objekts ermöglicht wird.

Durch die Verwendung des Bayer-Filters wird also zusätzlich zur dreidimensionalen Vermessung des Objekts die Farbmessung ermöglicht.

Vorteilhafterweise kann unter Verwendung der ersten Aufnahme in der ersten Sensorposition und der zweiten Aufnahme in der zweiten Sensorposition, die um einen Pixel relativ zur erstes Sensorposition senkrecht zu den Streifen versetzt ist, für jeden Pixel des Sensors ein erster Intensitätswert in der ersten Sensorposition und ein zweiter Intensitätswert in der zweiten Sensorposition ermittelbar sein, wobei mittels einer Recheneinheit der Kamera durch Differenzbildung ein Differenzwert zwischen dem ersten Intensitätswert und dem zweiten Intensitätswert ermittelbar ist, wobei unter Verwendung des Differenzwertes in Abhängigkeit vom Fokusabstand für jeden Pixel eine Tiefeninformation einer Objektoberfläche des Objekts mittels der Recheneinheit ermittelt wird, so dass auf diese Weise dreidimensionale Oberflächendaten des Objekts messbar sind.

Dadurch wird auf eine einfache Art und Weise die Bestimmung des Differenzwertes als ein Maß für die Änderung des Kontrastes ermöglicht. Im Maximum der Änderung des Differenzwertes liegt also die scharfe Ebene in der Objektoberfläche.

Vorteilhafterweise kann der Sensor ein CMOS-Sensor oder ein CCD-Sensor sein.

Dadurch können die herkömmlichen Sensor-Arten verwendet werden. Ein CMOS-Sensor hätte den Vorteil, dass die einzelnen Aufnahmen schneller ausgelesen werden können.

Vorteilhafterweise kann die oszillierende Bewegung des Sensors mittels eines Elektormotors oder mittels eines Piezzoelements mit einer Frequenz von mindestens 3000 Hz erfolgen.

Eine Frequenz in der Höhe von 6000 Hz ergibt sich beispielsweise bei einer Kamera, die pro Tiefenscan 300 Scanpositionen aufweist, wobei in jeder Scanposition zwei Aufnahmen in den beiden Sensorpositionen erfolgen, wobei die Aufnahmen einer Aufnahmezeit von 100 ms aufweisen. Bei einer geringeren Anzahl von Scanpositionen und bei längeren Aufnahmezeiten kann die Frequenz auch deutlich niedriger sein und beispielsweise bis zu 100 Hz aufweisen.

Ein Piezzoelements ist besonders gut geeignet, um eine besonders schnelle und präzise Verstellung zwischen den beiden Scanpositionen zu ermöglichen.

Die Erfindung betrifft weiterhin ein Verfahren zur dreidimensionalen Vermessung eines dentalen Objekts mittels einer Kamera, aufweisend mindestens eine Lichtquelle, die einen Beleuchtungsstrahl abstrahlt, mindestens ein Projektionsmittel, das ein Projektionsmuster erzeugt, eine Fokussierungsoptik, die das Projektionsmuster in einer scharfen Ebene in einem festgelegten Fokusabstand relativ zur dentalen Kamera abbildet, wobei das auf das Objekt projizierte Projektionsmuster als ein Beobachtungsstrahl vom Objekt zurückgestrahlt wird und mittels eines Sensors aufgenommen wird. Bei der Vermessung des Objekts wird die Fokussierungsoptik so gesteuert, dass der Fokusabstand der scharfen Ebene relativ zu Kamera schrittweise zwischen mehreren festgelegten Scanpositionen verändert wird, wobei für jede Scanposition mittels des Sensors eine erste Aufnahme und mindestens eine zweite Aufnahme erfolgt.

Dadurch werden also für jede Scanposition mindestens zwei Aufnahmen erzeugt, um den Kontrast zu bestimmen.

Ein Vorteil dieses Verfahrens liegt darin, dass auf eine einfache Art und Weise durch Vergleich der beiden Aufnahmen ein Kontrast bzw. die Schärfe der beiden Bilder bestimmt werden kann, um daraus den Abstand des Objekts zur Kamera und damit die dreidimensionalen Bilddaten des Objekts zu bestimmen.

Vorteilhafterweise kann der Sensor seitlich zum Strahlengang des Beobachtungsstrahls oszillierend hin und her bewegt werden, wobei die erste Aufnahme in einer ersten Sensorposition des Sensors und die zweite Aufnahme in einer zweiter Sensorposition des Sensors aufgenommen wird, wobei der Sensor bei der oszillierenden Bewegung um einen Abstand zwischen der ersten Sensorposition und der zweiten Sensorposition entlang einer ersten Sensorachse parallel zu den Zeilen den Sensorpixel oder entlang einer zweiten Sensorachse parallel zu den Spalten der Sensorpixel versetzt wird, der der Breite eines Pixels des Sensors entspricht.

Dadurch wird durch die Bewegung des Sensors jedes Pixel abwechselnd mit einem hellen Musterelement oder einem dunklen Musterelement des Projektionsmusters beleuchtet. Auf diese Weise lässt sich durch die zeitliche Abhängigkeit der Intensitäten jedes Pixels der Differenzwert und damit der Kontrast ermitteln.

Vorteilhafterweise kann das Projektionsmittel seitlich zum Strahlengang des Beleuchtungsstrahls oszillierend hin und her bewegt werden, wobei die erste Aufnahme in einer ersten Position des Projektionsmittels und die zweite Aufnahme in einer zweiter Position des Projektionsmittels aufgenommen wird, wobei das Projektionsmittel bei der oszillierenden Bewegung um einen Abstand versetzt wird, der so bemessen ist, dass das Projektionsmuster in der Ebene des Sensors um die Breite eines Pixels des Sensors entlang einer ersten Sensorachse parallel zu den Zeilen oder entlang einer zweiten Sensorachse parallel zu den Spalten versetzt wird.

Dadurch wird die Änderung des Projektionsmusters durch die oszillierende Bewegung des Projektionsmittels erzeugt.

Vorteilhafterweise kann das Projektionsmuster ein schachbrettförmiges Muster sein und aus dunklen und hellen quadratischen Musterelementen bestehen.

Dadurch wird beim Versetzen des Projektionsmusters um einen Pixel auf jedes Pixel des Sensors abwechselnd ein helles oder ein dunkles Musterelement projiziert.

Vorteilhafterweise kann das Projektionsmittel so bemessen und ausgerichtet sein, dass jedes Musterelement des Projektionsmusters auf einen Pixel des Sensors projiziert wird, so dass das projizierte Bild des Musterelements in der Ebene des Sensors den Abmessungen des Pixels entspricht.

Dadurch ist die Bewegung des Projektionsmittels minimal und kann mittels eines kompakten und präzisen Antriebs, wie eines Piezoelements, gewährleistet werden.

Vorteilhafterweise kann die Kamera eine Beobachtungsmaske im Strahlengang des Beobachtungsstrahls vor dem Sensor aufweisen, die mit dem Sensor mitbewegt wird, so dass die Abmessungen einer Abbildung eines Musterelements des Projektionsmusters in der Ebene der Beobachtungsmaske den Abmessungen eines Beobachtungsmaskenelements entsprechen.

Dadurch entspricht die Beobachtungsmaske in ihrer Struktur dem Projektionsmuster.

Vorteilhafterweise kann das Projektionsmuster aus mehreren parallelen Streifen bestehen.

Dadurch kann zur Erzeugung des Projektionsmusters beispielsweise ein herkömmliches Projektionsgitter mit parallelen Streifen verwendet werden.

Vorteilhafterweise kann das Projektionsmittel so bemessen und ausgerichtet sein, dass jedes Streifen des Projektionsmusters auf eine Spalte oder eine Zeile von Pixeln des Sensors projiziert wird, so dass die Breite eines projizierten Streifens in der Ebene des Sensors der Breite des Pixels entspricht, wobei der Sensor bzw. das Projektionsmittel senkrecht zu den projizierten Streifen bewegt wird.

Dadurch wird durch das Versetzen des Projektionsmusters um eine Pixelbreite auf jedes Pixel des Sensors abwechselnd ein heller oder ein dunkler Streifen projiziert.

Vorteilhafterweise kann unter Verwendung der ersten Aufnahme in der ersten Sensorposition bzw. in der ersten Position des Projektionsmittels und der zweite Aufnahme in der zweiten Sensorposition bzw. in der zweiter Position des Projektionsmittels, die um einen Pixel relativ zur erstes Sensorposition versetzt ist, für jeden Pixel des Sensors ein erster Intensitätswert in der ersten Sensorposition bzw. in der ersten Position des Projektionsmittels und ein zweiter Intensitätswert in der zweiten Sensorposition bzw. in der zweiten Position des Projektionsmittels ermittelt werden, wobei mittels einer Recheneinheit der Kamera durch Differenzbildung ein Differenzwert zwischen dem ersten Intensitätswert und dem weiten Intensitätswert ermittelt wird.

Dadurch kann der Differenzwert als ein Maß für den Kontrast auf eine einfache Art und Weise durch das Versetzen des Streifenmusters ermittelt werden.

Vorteilhafterweise kann unter Verwendung des Differenzwertes in Abhängigkeit vom Fokusabstand für jeden Pixel eine Tiefeninformation einer Objektoberfläche des Objekts mittels der Recheneinheit ermittelt werden, so dass auf diese Weise dreidimensionale Oberflächendaten des Objekts erzeugt werden.

Dadurch wird also für jeden Pixel der Differenzwert in Abhängigkeit vom Fokusabstand ermittelt wobei das Maximum des Differenzwertes der Position der Objektoberfläche entspricht.

Vorteilhafterweise kann die Kamera eine Beobachtungsmaske im Strahlengang des Beobachtungsstrahls vor dem Sensor aufweisen, die zusammen mit dem Sensor mitbewegt wird, wobei die Beobachtungsmaske ein Bayer-Filter ist, der aus einer schachbrettförmigen Struktur aus roten, grünen und blauen Farbfiltern besteht, die jeweils einem Pixel des Sensors zugeordnet sind, so dass eine Farbmessung des dentalen Objekts ermöglicht wird.

Dadurch wird also zusätzlich zur dreidimensionalen Vermessung des Objekts die Farbmessung des Objekts ermöglicht. Eine quadratische Vierer-Gruppe kann beispielsweise aus zwei blauen, einem grünen und einem roten Farbfilter bestehen.

Vorteilhafterweise kann die oszillierende Bewegung des Sensors bzw. des Projektionsmittels mittels eines Elektormotors oder mittels eines Piezzoelements mit einer Frequenz von mindestens 6000 Hz erfolgen.

### Kurzbeschreibung der Zeichnungen

Die Erfindung wird anhand der Zeichnungen erläutert. Es zeigt, die
- Fig. 1: eine Skizze einer Kamera zur dreidimensionalen Vermessung, die
- Fig. 2: eine Skizze eines schachbrettförmigen Projektionsmusters, die
- Fig. 3: eine Skizze eines Projektionsmusters, der aus dunklen Streifen und hellen Streifen besteht, die
- Fig .4: ein Diagramm eines Intensitätswertes in Abhängigkeit von der Zeit, die
- Fig. 5: ein Diagramm eines Differenzwertes in Abhängigkeit von der Zeit, die
- Fig .6: ein Diagramm des Differenzwertes in Abhängigkeit von einem Fokusabstand. Ausführungsbeispiele

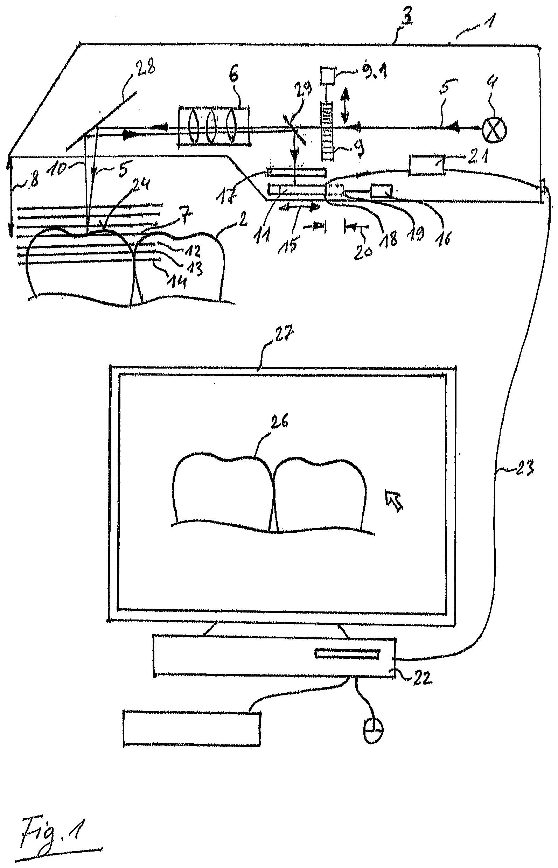

Die Fig. 1 zeigt eine Skizze einer Kamera 1 zur dreidimensionalen Vermessung eines dentalen Objekts 2, wie der dargestellten Zähne eines Patienten, wobei die Kamera 1 in ein Gehäuse 3 in Form eines Handstücks integriert ist. Die Kamera weist eine Lichtquelle 4 auf, die einen Beleuchtungsstrahl 5 abstrahlt, eine Fokussierungsoptik zur Fokussierung des Beleuchtungsstrahls 5 auf eine scharfe Ebene 7 in einem festgelegten Fokusabstand 8 relativ zur Kamera 1. Die Kamera 1 weist darüber hinaus ein Projektionsmittel 9, wie ein Projektionsgitter oder ein LCD-Lichtprojektor, zur Erzeugung eines Projektionsmusters auf. Das Projektionsmuster kann dabei beispielsweise eine schachbrettförmige Form aufweisen oder aus mehreren parallelen Streifen bestehen. Das Projektionsmuster wird also auf das Objekt 2 projiziert und als ein Beobachtungsstrahl 10 vom Objekt 2 zurückgestrahlt und mittels eines Sensors 11 aufgenommen. Bei der Vermessung des Objekts 2 wird die Fokussierungsoptik 6, die beispielsweise aus mehreren Linsen bestehen kann, so angesteuert, dass der Fokusabstand 8 der scharfen Ebene 7 relativ zur Kamera 2 schrittweise zwischen mehreren festgelegten Scanpositionen 12, 13 und 14 verstellt wird. Für jede Scanposition 7, 12, 13 oder 14 wird eine erste Aufnahme und mindestens eine zweite Aufnahme erzeugt, wobei der Sensor 11 seitlich zum Strahlengang des Beobachtungsstrahls 10, wie durch den Pfeil 15 angedeutet ist, mittels eines Antriebsmittels 16, wie eines Motors oder eines Piezoelements, bewegt wird. Durch die oszillierende Bewegung des Sensors 11 wird also in jeder Scanposition 12, 13 und 14 jeder Pixel des Sensors 11 abwechselnd mit einem hellen oder einem dunklen Musterelement des Projektionsmusters beleuchtet wird. Die Scanpositionen 7, 12, 13 und 14 können beispielsweise in einem Abstand von 0,1 mm zueinander angeordnet sein. Die Auflösung in der Z-Richtung parallel zum Beobachtungsstrahl 10 ist damit durch diesen Abstand zwischen den Scanpositionen gegeben. Die Kamera weist zusätzlich eine Beobachtungsmaske 17 auf, die beispielsweise in Form eines Bayer-Filters aus mehreren Farbfiltern ausgeführt sein kann. Ein solcher Bayer-Filter 17 ermöglicht zusätzlich zur dreidimensionalen Vermessung des Objekts 2 eine Farbmessung des Objekts 2. Bei der Bewegung 15 des Sensors 11 wird der Sensor 11 zwischen einer ersten Sensorposition 18 und einer zweiten Sensorposition 19, die gestrichelt dargestellt ist, um eine Abstand 20 verschoben. Dieser Abstand 20 kann beispielsweise der Breite eines Pixels des Sensors 11 entsprechen. Dadurch wird also für jeden Pixel des Sensors 11 ein erster Intensitätswert in der ersten Sensorposition 18 und ein zweiter Intensitätswert in den zweiten Sensorposition 19 bestimmt, wobei mittels einer Recheneinheit 21 durch Differenzbildung ein Differenzwert zwischen dem ersten Intensitätswert und dem zweiten Intensitätswert ermittelt wird. Die Beobachtungsmaske 17 kann beispielsweise zusammen mit dem Sensor 11 zwischen beiden Sensorpositionen 18 und 19 mitbewegt werden.

Die Recheneinheit 21 kann beispielsweise ein Mikrocomputer oder ein Chip sein, der in die Kamera 1 integriert ist. Alternativ kann die Ermittlung des Differenzwertes mittels eines Computers 22 erfolgen, wobei die Bilddaten des Sensors 11 mittels einer Kabelverbindung 23 oder kabellos an den Computer übermittelt werden. Auf diese Weise wird also für jeden Pixel und für jede Scanposition 7, 12, 13 oder 14 ein Differenzwert ermittelt, wobei der Differenzwert im Maximum ist, falls die scharfe Schicht 7 mit einer Oberfläche 24 des Objekts 2 übereinstimmt. Andernfalls wird das Projektionsmuster unscharf auf der Oberfläche 24 des Objekts 2 abgebildet. Die Bilddaten des Sensors 11 werden also nach jeder Aufnahme ausgelesen, wie durch den Pfeil 25 dargestellt ist und an den Computer übermittelt. Die einzelnen Koordinaten der Messpunkte auf der Oberfläche 24 des Messobjekts 2 werden nach der vollständigen Vermessung des Objekts 2 verwendet, um dreidimensionale Bilddaten 26 des Objekts 2 zu berechnen, die mittels einer Anzeigevorrichtung 27, wie eines Monitors dargestellt werden können. In einem Beispiel, das nicht zur Erfindung gehört, kann zur Verstellung des Sensors 11 auch das Projektionsmittel 9 unter Verwendung eines zweiten Antriebsmittels 9.1, wie eines Elektromotors oder eines Piezoelements, angetrieben werden. Die Ansteuerung des Projektionsmittels 9 erfolgt dann so, dass in der Ebene des Sensors 11 das Projektionsmuster um die Breite eines Pixels verschoben wird, wie durch den Pfeil angedeutet ist. Bei einer weiteren Alternative kann sowohl der Sensor 11 als auch das Projektionsmittel 9 synchron verschoben werden, um die gewünschte Verschiebung des Projektionsmusters relativ zum Sensor 11 zu erzeugen. Die Kamera 1 weist einen Strahlumlenker 28, wie einen Spiegel auf, der den Beleuchtungsstrahl 5 zum Objekt 2 umlenkt. Darüber hinaus weist die Kamera 1 einen Strahlteiler 29 auf, die den Beobachtungsstrahl 10 zum Sensor 11 umlenkt. Die Fokussierungsoptik 6 wird sowohl vom Beleuchtungsstrahl 5 als auch vom Beobachtungsstrahl 10 durchstrahlt.

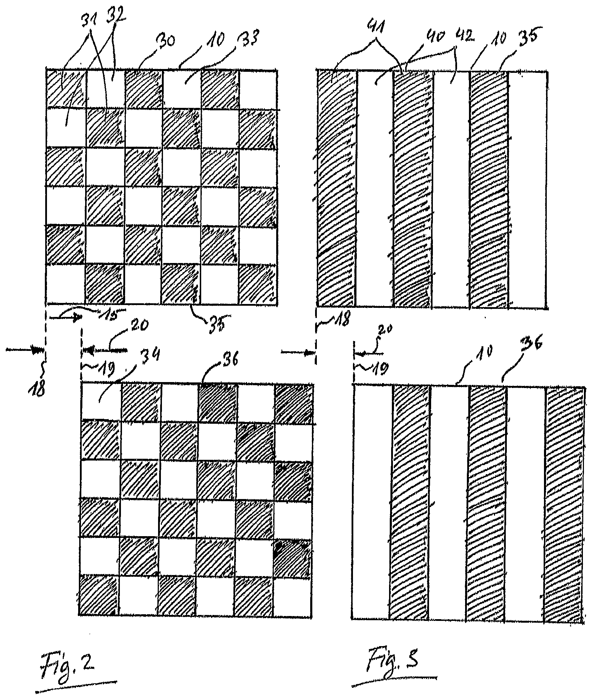

Die Fig. 2 zeigt eine Skizze eines schachbrettförmigen Projektionsmusters 30 auf dem Sensor 11 in der ersten Sensorposition 18. Die dunklen Musterelemente 31 des Projektionsmusters 30 sowie hellen Musterelemente 32 des Projektionsmusters 30 entsprechen in den Abmessungen den einzelnen Pixeln 33 des Sensors 11. Durch die seitliche Verschiebung 15 um den Abstand 20, der der Breite eines Pixels 33 entspricht, wird also beispielsweise das Pixel 34 in der linken oberen Ecke mit einem hellen Musterelement 32 beleuchtet. Auf diese Weise wird jedes Pixel des Sensors 11 abwechselnd mit einem hellen Musterelement 32 oder einem dunklen Musterelement 31 beleuchtet. Dadurch lässt sich also aus der ersten Aufnahme 35 in der Sensorposition 18 und aus der zweiten Aufnahme 36 in der zweiten Sensorposition 19 für jedes Pixel ein Differenzwert der beiden Intensitätswerte ermittelt. Falls die scharfe Schicht mit dem Objektoberfläche 24 des Objekts übereinstimmt, wird das Projektionsmuster 30 scharf auf den Sensor 11 abgebildet, so dass die einzelnen Differenzwerte im Maximum liegen.

Die Fig. 3 zeigt eine Skizze eines Projektionsmusters 40, der aus dunklen Streifen 41 und hellen Streifen 42 besteht. Das Projektionsmuster wird sich wie in Fig. 2, zwischen der ersten Sensorposition 18 und der zweiten Sensorposition 19 um den Abstand 20 verschoben, der einer Pixelbreite entspricht. Durch diese Verschiebung des Sensors 11 wird jeder Pixel des Sensors 11 ebenfalls abwechselnd mit einem hellen Streifen 42 oder einem dunklen Streifen 41 beleuchtet.

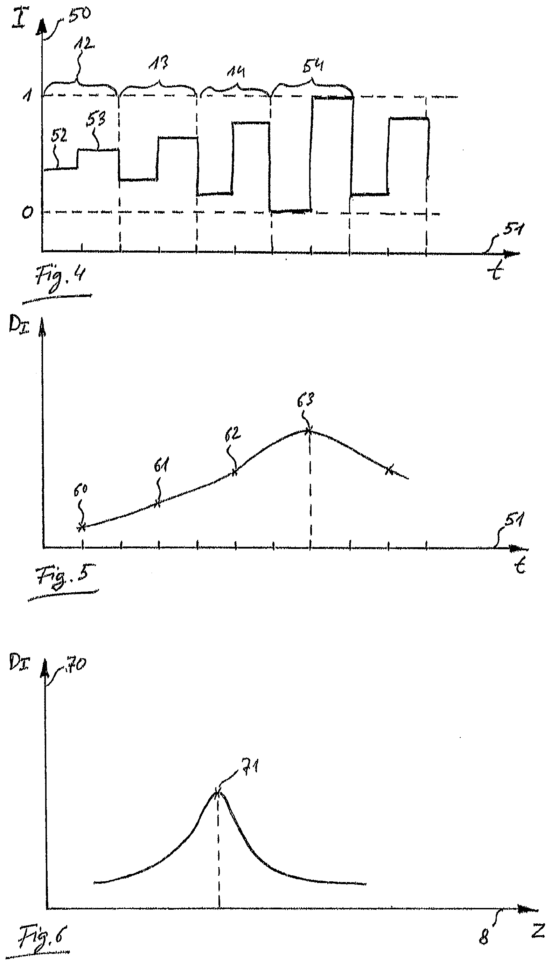

Die Fig. 4 zeigt ein Diagramm eines Intensitätswertes 50 auf der Y-Achse in Abhängigkeit von der Zeit 51 auf der X-Achse. Dabei wird in einer ersten Scanposition 12 ein erster Intensitätswert 52 auf einer Skala zwischen 0 und 1 aus der ersten Aufnahme 35 in der ersten Sensorposition 18 und ein zweiter Intensitätswert 53 aus der zweiten Aufnahme 36 in der zweiten Sensorposition 19 ermittelt. Die Intensitätswerte werden entsprechend für eine zweite Scanposition 13, eine dritte Scanposition 14 und eine fünfte Scanposition 54 ermittelt.

Die Fig. 5 zeigt ein Diagramm zur Verdeutlichung des vorliegenden Verfahrens, wobei aus dem Intensitätswert 52 und 53 durch Differenzbildung ein erster Differenzwert 60 für die erste Scanposition 12, ein zweiter Differenzwert 61 für die zweite Scanposition 13, ein dritter Differenzwert 62 für die dritte Scanposition 14 und ein vierter Differenzwert 63 für die vierte Scanposition 54 ermittelt werden. Die Differenzwerte sind in Abhängigkeit von der Zeit 51 aufgetragen. In der vierten Scanposition 54 ist der Differenzwert 63 maximal, so dass in dieser Scanposition 54 die scharfe Ebene 7 mit der Oberfläche 24 des Objekts übereinstimmt. Dadurch lässt sich also für jedes Pixel eine Tiefeninformation des entsprechenden Messpunktes auf der Oberfläche 24 des Objekts ermitteln.

Die Fig. 6 zeigt den Differenzwert 70 in Abhängigkeit vom Fokusabstand 8 für einen einzelnen Pixel des Sensors 11. In einem Maximum 71 des Differenzwertes ist der Kontrast maximal, da das Projektionsmuster 30 aus Fig. 2 scharf abgebildet wird.

### Bezugszeichen

- 1: Kamera
- 2: Objekt
- 3: Gehäuse
- 4: Lichtquelle
- 5: Beleuchtungsstrahl
- 6: Fokussierungsoptik
- 7: scharfe Ebene
- 8: Fokusabstand
- 9: Projektionsmittel
- 9.1: Antriebsmittel
- 10: Beobachtungsstrahl
- 11: Sensor
- 12: Scanposition
- 13: Scanposition
- 14: Scanpostion
- 15: Pfeil
- 16: Antriebsmittel
- 17: Beobachtungsmaske/Bayer-Filter
- 18: Sensorposition
- 19: Sensorposition
- 20: Abstand
- 21: Recheinheit
- 22: Computer
- 23: Kabelverbindung
- 24: Oberfläche
- 25: Pfeil
- 26: Bilddaten
- 27: Anzeigevorrichtung
- 28: Spiegel
- 29: Strahlteiler
- 30: Projektionsmuster
- 31: Musterelement
- 32: Musterelement
- 33: Pixel
- 34: Pixel
- 35: erste Aufnahme
- 36: zweite Aufnahme
- 40: Projektionsmuster
- 41: dunkle Streifen
- 42: helle Streifen
- 50: Intensitätswert
- 51: Zeit
- 52: Intensitätswert
- 53: Intensitätswert
- 54: Scanposition
- 60: erster Differenzwert
- 61: zweiter Differenzwert
- 62: dritter Differenzwert
- 63: vierter Differenzwert
- 70: Differenzwert
- 71: Maximum

## Patentansprüche

1. Kamera (1) zur dreidimensionalen Vermessung eines dentalen Objekts (2), aufweisend mindestens eine Lichtquelle (4), die einen Beleuchtungsstrahl (5) abstrahlt, mindestens ein Projektionsmittel (9), das ein Projektionsmuster (30, 40) erzeugt, eine Fokussierungsoptik (6), die das Projektionsmuster (30, 40) in einer scharfen Ebene (7) in einem festgelegten Fokusabstand (8) relativ zur dentalen Kamera (1) abbildet, wobei das auf das Objekt (2) projizierte Projektionsmuster (30, 40) als ein Beobachtungsstrahl (10) vom Objekt (2) zurückgestrahlt wird und mittels eines Sensors (11) aufgenommen wird, eine Steuerung für die Fokussierungsoptik, die eingerichtet ist, bei der Vermessung des Objekts (2) die Fokussierungsoptik (6) so zu steuern, dass der Fokusabstand (8) der scharfen Ebene (7) relativ zur Kamera (1) schrittweise zwischen mehreren festgelegten Scanpositionen (12, 13, 14, 54) verändert wird, wobei der Sensor eingerichtet ist, für jede Scanposition (12, 13, 14, 54) eine erste Aufnahme (35) und mindestens eine zweite Aufnahme (36) zu erzeugen, Mittel um den Sensor (11) seitlich zum Strahlengang des Beobachtungsstrahls oszillierend hin und her zu bewegen, wobei der Sensor eingerichtet ist, die erste Aufnahme (35) in einer ersten Sensorposition (18) des Sensors (11) und die zweite Aufnahme (36) in einer zweiten Sensorposition (19) des Sensors (11) aufzunehmen.

2. Kamera (1) nach Anspruch 1, wobei das Projektionsmuster (30) ein schachbrettförmiges Muster ist und aus dunklen und hellen quadratischen Musterelementen (31, 32) besteht.

3. Kamera (1) nach Anspruch 2, wobei das Projektionsmittel (9) so bemessen und ausgerichtet ist, dass jedes Musterelement (31, 32) des Projektionsmusters (30, 40) auf einen Pixel (33, 34) des Sensors (11) projiziert wird, so dass das projizierte Bild des Musterelements (31, 32) in der Ebene (7) des Sensors (11) den Abmessungen des Pixels (33, 34) entspricht.

4. Kamera (1) nach Anspruch 3, wobei die Mittel zur Bewegung des Sensors so eingerichtet sind, dass der Sensor bei der oszillierenden Bewegung um einen Abstand (20) zwischen der ersten Sensorposition (18) und der zweiten Sensorposition (19) versetzt wird, der der Breite eines Pixels (33, 34) des Sensors (11) entspricht.

5. Kamera (1) nach Anspruch 4, wobei die Mittel zur Bewegung des Sensors so eingerichtet sind, dass der Sensor (11) entlang einer ersten Sensorachse parallel zu den Zeilen oder entlang einer zweiten Sensorachse parallel zu den Spalten bewegt wird.

6. Kamera (1) nach einem der Ansprüche 1 bis 5, wobei die Kamera (1) eine Beobachtungsmaske (17) im Strahlengang des Beobachtungsstrahls vor dem Sensor (11) aufweist, die so eingerichtet ist, dass sie zusammen mit dem Sensor (11) zwischen den beiden Sensorpositionen (18, 19) mitbewegt wird.

7. Kamera (1) nach Anspruch 6, wobei die Beobachtungsmaske (17) ein Bayer-Filter ist, der aus einer schachbrettförmigen Struktur aus roten, grünen und blauen Farbfiltern besteht, die jeweils einem Pixel (33, 34) des Sensors (11) zugeordnet sind, so dass eine Farbmessung des dentalen Objekts (2) ermöglicht wird.

8. Kamera (1) nach einem der Ansprüche 1 bis 7, die eingerichtet ist unter Verwendung der ersten Aufnahme (35) in der ersten Sensorposition (18) und der zweiten Aufnahme (36) in der zweiten Sensorposition (19), die um einen Pixel (33, 34) relativ zur ersten Sensorposition (18) versetzt ist, für jeden Pixel (33, 34) des Sensors (11) einen ersten Intensitätswert (52) in der ersten Sensorposition (18) und einen zweiten Intensitätswert (53) in der zweiten Sensorposition (19) zu ermitteln, wobei die Kamera (1) eine Recheneinheit (21) umfasst, die eingerichtet ist, durch Differenzbildung ein Differenzwert (60, 61, 62, 63, 70) zwischen dem ersten Intensitätswert (52) und dem zweiten Intensitätswert (53) zu ermitteln.

9. Kamera (1) nach Anspruch 8, die eingerichtet ist unter Verwendung des Differenzwertes (60, 61, 62, 63, 70) in Abhängigkeit vom Fokusabstand (8, 20) für jeden Pixel (33, 34) eine Tiefeninformation einer Objektoberfläche (24) des Objekts (2) mittels der Recheneinheit (21) zu ermitteln, so dass auf diese Weise dreidimensionale Oberflächendaten (26) des Objekts (2) messbar sind.

10. Kamera (1) nach Anspruch 1, wobei das Projektionsmuster (40) aus mehreren parallelen hellen und dunklen Streifen (41) besteht.

11. Kamera (1) nach Anspruch 10, wobei das Projektionsmittel (9) so bemessen und ausgerichtet ist, dass jeder Streifen (41, 42) des Projektionsmusters (40) auf eine Spalte oder eine Zeile von Pixeln (33, 34) des Sensors (11) projiziert wird, so dass die Breite eines projizierten Streifens (41, 42) in der Ebene (7) des Sensors (11) der Breite des Pixels (33, 34) entspricht.

12. Kamera (1) nach Anspruch 10 oder 11, die eingerichtet ist, unter Verwendung der ersten Aufnahme (35) in der ersten Sensorposition (18) und der zweiten Aufnahme (36) in der zweiten Sensorposition (19), die um einen Pixel (33, 34) relativ zur ersten Sensorposition (18) senkrecht zu den Streifen (41, 42) versetzt ist, für jeden Pixel (33, 34) des Sensors (11) einen ersten Intensitätswert (52) in der ersten Sensorposition (18) und einen zweiten Intensitätswert (53) in der zweiten Sensorposition (19) zu ermitteln, wobei die Recheneinheit (21) eingerichtet ist, durch Differenzbildung ein Differenzwert (60, 61, 62, 63, 70) zwischen dem ersten Intensitätswert (52) und dem zweiten Intensitätswert (53) zu ermitteln, und unter Verwendung des Differenzwertes (60, 61, 62, 63, 70) in Abhängigkeit vom Fokusabstand (8, 20) für jeden Pixel (33, 34) eine Tiefeninformation einer Objektoberfläche des Objekts (2) zu ermitteln, so dass auf diese Weise dreidimensionale Oberflächendaten des Objekts (2) messbar sind.

13. Kamera (1) nach einem der Ansprüche 1 bis 12, wobei die oszillierende Bewegung des Sensors (11) mittels eines Elektormotors oder mittels eines Piezzoelements mit einer Frequenz von mindestens 6000 Hz erfolgt.

14. Verfahren zur dreidimensionalen Vermessung eines dentalen Objekts (2) mittels einer Kamera (1), aufweisend mindestens eine Lichtquelle (4), die einen Beleuchtungsstrahl (5) abstrahlt, mindestens ein Projektionsmittel (9), das ein Projektionsmuster (30, 40) erzeugt, eine Fokussierungsoptik (6), die das Projektionsmuster (30, 40) in einer scharfen Ebene (7) in einem festgelegten Fokusabstand (8) relativ zur dentalen Kamera (1) abbildet, wobei das auf das Objekt (2) projizierte Projektionsmuster (30, 40) als ein Beobachtungsstrahl vom Objekt (2) zurückgestrahlt wird und mittels eines Sensors (11) aufgenommen wird, wobei bei der Vermessung des Objekts (2) die Fokussierungsoptik (6) so gesteuert wird, dass der Fokusabstand (8) der scharfen Ebene (7) relativ zu Kamera (1) schrittweise zwischen mehreren festgelegten Scanpositionen (12, 13, 14, 54) verändert wird, wobei für jede Scanposition (12, 13, 14, 54) mittels des Sensors (11) eine erste Aufnahme (35) und mindestens eine zweite Aufnahme (36) erfolgt, wobei der Sensor (11) seitlich zum Strahlengang des Beobachtungsstrahls oszillierend hin und her bewegt wird, wobei die erste Aufnahme (35) in einer ersten Sensorposition (18) des Sensors (11) und die zweite Aufnahme (36) in einer zweiter Sensorposition (19) des Sensors (11) aufgenommen wird, wobei der Sensor (11) bei der oszillierenden Bewegung um einen Abstand (20) zwischen der ersten Sensorposition (18) und der zweiten Sensorposition (19) entlang einer ersten Sensorachse parallel zu den Zeilen den Sensorpixel oder entlang einer zweiten Sensorachse parallel zu den Spalten der Sensorpixel versetzt wird, der der Breite eines Pixels (33, 34) des Sensors (11) entspricht.

15. Verfahren nach Anspruch 14, wobei das Projektionsmuster (30) ein schachbrettförmiges Muster ist und aus dunklen und hellen quadratischen Musterelementen (31, 32) oder aus mehreren parallelen Streifen (41, 42) besteht.

## Claims

1. A camera (1) for the three-dimensional measurement of a dental object (2), having at least one light source (4) that emits an illuminating beam (5), at least one projection means (9) that generates a projection pattern (30, 40), focusing optics (6) that display the projection pattern (30, 40) in a plane of sharp focus (7) at a defined focal distance (8) relative to the dental camera (1), wherein the projection pattern (30, 40) projected onto the object (2) is reflected by the object (2) as an observation beam (10) and is acquired by means of a sensor (11), a controller for the focusing optics which is configured to control the focusing optics (6) during the measurement of the object (2) so that the focal distance (8) of the plane of sharp focus (7) relative to the camera (1) is changed incrementally between several defined scan positions (12, 13, 14, 54), wherein the sensor is configured to produce a first image (35) and at least one second image (36) for each scan position (12, 13, 14, 54), means for moving the sensor (11) to and fro in an oscillating manner, laterally with respect to the beam path of the observation beam, wherein the sensor is configured to take the first image (35) in a first sensor position (18) of the sensor (11) and the second image (36) in a second sensor position (19) of the sensor (11).

2. The camera (1) according to claim 1, wherein the projection pattern (30) is a chessboard-like pattern and consists of dark and light square pattern elements (31, 32).

3. The camera (1) according to claim 2, wherein the projection means (9) is dimensioned and oriented such that each pattern element (31, 32) of the projection pattern (30, 40) is projected onto a pixel (33, 34) of the sensor (11) so that the projected image of the pattern element (31, 32) in the plane (7) of the sensor (11) corresponds to the dimensions of the pixel (33, 34).

4. The camera (1) according to claim 3, wherein the means for moving the sensor are configured such that, during the oscillating movement, the sensor is displaced by a distance (20) between the first sensor position (18) and the second sensor position (19) that corresponds to the width of a pixel (33, 34) of the sensor (11).

5. The camera (1) according to claim 4, wherein the means for moving the sensor are configured such that the sensor (11) is moved along a first sensor axis parallel to the rows or along a second sensor axis parallel to the columns.

6. The camera (1) according to any one of claims 1 to 5, wherein the camera (1) has an observation mask (17) in the beam path of the observation beam in front of the sensor (11), which mask is configured such that it is moved together with the sensor (11) between the two sensor positions (18, 19).

7. The camera (1) according to claim 6, wherein the observation mask (17) is a Bayer filter which consists of a chessboard-like structure of red, green and blue colour filters, each of which is associated with one pixel (33, 34) of the sensor (11), so that a colour measurement of the dental object (2) is facilitated.

8. The camera (1) according to any one of claims 1 to 7, which is configured to determine, using the first image (35) in the first sensor position (18) and the second image (36) in the second sensor position (19), which is offset by one pixel (33, 34) relative to the first sensor position (18), for each pixel (33, 34) of the sensor (11), a first intensity value (52) in the first sensor position (18) and a second intensity value (53) in the second sensor position (19), wherein the camera (1) comprises a computing unit (21) which is configured to determine a difference value (60, 61, 62, 63, 70) by calculating the difference between the first intensity value (52) and the second intensity value (53).

9. The camera (1) according to claim 8, which is configured to determine by means of the computing unit (21), using the difference value (60, 61, 62, 63, 70) as a function of the focal distance (8, 20), depth information of an object surface (24) of the object (2) for each pixel (33, 34), so that three-dimensional surface data (26) of the object (2) can be measured in this way.

10. The camera (1) according to claim 1, wherein the projection pattern (40) consists of a plurality of parallel light and dark stripes (41).

11. The camera (1) according to claim 10, wherein the projection means (9) is dimensioned and oriented such that each stripe (41, 42) of the projection pattern (40) is projected onto a column or a row of pixels (33, 34) of the sensor (11) so that the width of a projected stripe (41, 42) in the plane (7) of the sensor (11) corresponds to the width of the pixel (33, 34).

12. The camera (1) according to claim 10 or 11, which is configured to determine, using the first image (35) in the first sensor position (18) and the second image (36) in the second sensor position (19), which is offset by one pixel (33, 34) relative to the first sensor position (18) perpendicular to the stripes (41, 42), for each pixel (33, 34) of the sensor (11) a first intensity value (52) in the first sensor position (18) and a second intensity value (53) in the second sensor position (19), wherein the computing unit (21) is configured to determine a difference value (60, 61, 62, 63, 70) by calculating the difference between the first intensity value (52) and the second intensity value (53), and to determine, using the difference value (60, 61, 62, 63, 70) as a function of the focal distance (8, 20), depth information of an object surface of the object (2) for each pixel (33, 34) so that three-dimensional surface data of the object (2) can be measured in this way.

13. The camera (1) according to any one of claims 1 to 12, wherein the oscillating movement of the sensor (11) is carried out by means of an electric motor or by means of a piezoelectric element at a frequency of at least 6,000 Hz.

14. A method for the three-dimensional measurement of a dental object (2) using a camera (1), having at least one light source (4) that emits an illuminating beam (5), at least one projection means (9) that generates a projection pattern (30, 40), focusing optics (6) that display the projection pattern (30, 40) in a plane of sharp focus (7) at a defined focal distance (8) relative to the dental camera (1), wherein the projection pattern (30, 40) projected onto the object (2) is reflected by the object (2) as an observation beam and is acquired by means of a sensor (11), wherein the focusing optics (6) are controlled during the measurement of the object (2) such that the focal distance (8) of the plane of sharp focus (7) relative to the camera (1) is changed incrementally between several defined scan positions (12, 13, 14, 54), wherein a first image (35) and at least one second image (36) is produced for each scan position (12, 13, 14, 54) by means of the sensor (11), wherein the sensor (11) is moved to and fro in an oscillating manner, laterally with respect to the beam path of the observation beam, wherein the first image (35) is taken in a first sensor position (18) of the sensor (11) and the second image (36) is taken in a second sensor position (19) of the sensor (11), wherein, during the oscillating movement, the sensor (11) is moved along a first sensor axis parallel to the rows of the sensor pixels or along a second sensor axis parallel to the columns of the sensor pixels by a distance (20) between the first sensor position (18) and the second sensor position (19) which corresponds to the width of a pixel (33, 34) of the sensor (11).

15. The method according to claim 14, wherein the projection pattern (30) is a chessboard-like pattern and consists of dark and light square pattern elements (31, 32) or of a plurality of parallel stripes (41, 42).

## Revendications

1. Caméra (1) de mesure tridimensionnelle d'un objet (2) dentaire, comportant au moins une source lumineuse (4), laquelle émet un faisceau d'éclairage (5), au moins un moyen de projection (9), lequel génère un motif de projection (30, 40), une optique de mise au point (6), laquelle reproduit le motif de projection (30, 40) dans un plan (7) net à une distance de mise au point (8) fixe par rapport à la caméra (1) dentaire, dans laquelle le motif de projection (30, 40) projeté sur l'objet (2) est réfléchi en tant qu'un faisceau d'observation (10) par l'objet (2) et est enregistré au moyen d'un capteur (11), une commande pour l'optique de mise au point, laquelle est conçue pour commander l'optique de mise au point (6) lors de la mesure de l'objet (2) de telle sorte que la distance de mise au point (8) du plan (7) net par rapport à la caméra (1) change progressivement entre plusieurs positions de balayage (12, 13, 14, 54) définies, dans laquelle le capteur est conçu pour générer pour chaque position de balayage (12, 13, 14, 54) un premier enregistrement (35) et au moins un second enregistrement (36), un moyen destiné au déplacement du capteur (11) de manière oscillante, en un mouvement de va-et-vient, latéralement au trajet du faisceau du faisceau d'observation, dans laquelle le capteur est conçu pour enregistrer le premier enregistrement (35) dans une première position de capteur (18) du capteur (11) et le second enregistrement (36) dans une seconde position de capteur (19) du capteur (11).

2. Caméra (1) selon la revendication 1, dans laquelle le motif de projection (30) est un motif en forme de damier et se compose d'éléments motifs carrés (31, 32) sombres et clairs.

3. Caméra (1) selon la revendication 2, dans laquelle le moyen de projection (9) est dimensionné et aligné de telle sorte que chaque élément motif (31, 32) du motif de projection (30, 40) soit projeté sur un pixel (33, 34) du capteur (11) de telle sorte que l'image projetée de l'élément motif (31, 32) dans le plan (7) du capteur (11) corresponde aux dimensions du pixel (33, 34).

4. Caméra (1) selon la revendication 3, dans laquelle le moyen destiné au déplacement du capteur est conçu de telle sorte que le capteur soit décalé pendant le mouvement oscillant d'une distance (20) entre la première position de capteur (18) et la seconde position de capteur (19), laquelle correspond à la largeur d'un pixel (33, 34) du capteur (11).

5. Caméra (1) selon la revendication 4, dans laquelle le moyen destiné au déplacement du capteur est conçu de telle sorte que le capteur (11) soit déplacé le long d'un premier axe de capteur parallèle aux rangées ou le long d'un second axe de capteur parallèle aux colonnes.

6. Caméra (1) selon l'une quelconque des revendications 1 à 5, dans laquelle la caméra (1) comporte un masque d'observation (17) dans le trajet de faisceau du faisceau d'observation devant le capteur (11), lequel est conçu pour se déplacer ensemble avec le capteur (11) entre les deux positions de capteur (18, 19).

7. Caméra (1) selon la revendication 6, dans laquelle le masque d'observation (17) est un filtre Bayer, lequel consiste en une structure en forme de damier de filtres de couleur rouge, vert et bleu, lesquels sont respectivement attribués à un pixel (33, 34) du capteur (11) de telle sorte qu'une mesure de la couleur de l'objet (2) dentaire soit rendue possible.

8. Caméra (1) selon l'une quelconque des revendications 1 à 7, laquelle est conçue pour déterminer à l'aide du premier enregistrement (35) dans la première position de capteur (18) et du second enregistrement (36) dans la seconde position de capteur (19), laquelle est décalé d'un pixel (33, 34) par rapport à la première position de capteur (18), pour chaque pixel (33, 34) du capteur (11), une première valeur d'intensité (52) dans la première position de capteur (18) et une seconde valeur d'intensité (53) dans la seconde position du capteur (19), dans laquelle la caméra (1) comprend une unité de calcul (21), laquelle est conçue pour déterminer, par formation de la différence, une valeur de différence (60, 61, 62, 63, 70) entre la première valeur d'intensité (52) et la seconde valeur d'intensité (53).

9. Caméra (1) selon la revendication 8, laquelle est conçue pour déterminer à l'aide de la valeur de différence (60, 61, 62, 63, 70) en fonction de la distance de mise au point (8, 20) pour chaque pixel (33, 34) des informations de profondeur d'une surface (24) de l'objet (2) au moyen de l'unité de calcul (21) de telle sorte que des données tridimensionnelles de surface (26) de l'objet (2) puissent être mesurées.

10. Caméra (1) selon la revendication 1, dans laquelle le motif de projection (40) consiste en une pluralité de bandes (41) parallèles claires et sombres.

11. Caméra (1) selon la revendication 10, dans laquelle le moyen de projection (9) est dimensionné et aligné de telle sorte que chaque bande (41, 42) du motif de projection (40) soit projetée sur une colonne ou sur une rangée de pixels (33, 34) du capteur (11) de telle sorte que la largeur d'une bande (41, 42) projetée dans le plan (7) du capteur (11) corresponde à la largeur du pixel (33, 34).

12. Caméra (1) selon la revendication 10 ou 11, laquelle est conçue pour déterminer à l'aide du premier enregistrement (35) dans la première position de capteur (18) et du second enregistrement (36) dans la seconde position de capteur (19), laquelle est décalée d'un pixel (33, 34) par rapport à la première position de capteur (18), perpendiculairement aux bandes (41, 42), pour chaque pixel (33, 34) du capteur (11), une première valeur d'intensité (52) dans la première position de capteur (18) et une seconde valeur d'intensité (53) dans la seconde position de capteur (19), dans laquelle l'unité de calcul (21) est conçue pour déterminer, par formation de la différence (53), une valeur de différence (60, 61, 62, 63, 70) entre la première valeur d'intensité (52) et la seconde valeur d'intensité, et pour déterminer, à l'aide de la valeur de différence (60, 61, 62, 63, 70) en fonction de la distance de mise au point (8, 20) pour chaque pixel (33, 34), des informations de profondeur d'une surface d'objet de l'objet (2) de telle sorte que des données tridimensionnelles de surface de l'objet (2) puissent être mesurées.

13. Caméra (1) selon l'une quelconque des revendications 1 à 12, dans laquelle le mouvement d'oscillation du capteur (11) s'effectue au moyen d'un moteur électrique ou au moyen d'un élément piézoélectrique présentant une fréquence d'au moins 6 000 Hz.

14. Procédé de mesure tridimensionnelle d'un objet (2) dentaire au moyen d'une caméra (1), comportant au moins une source lumineuse (4), laquelle émet un faisceau d'éclairage (5), au moins un moyen de projection (9), lequel génère un motif de projection (30, 40), une optique de mise au point (6), laquelle reproduit le motif de projection (30, 40) dans un plan (7) net à une distance de mise au point (8) fixe par rapport à la caméra (1) dentaire, dans lequel le motif de projection (30, 40) projeté sur l'objet (2) est réfléchi en tant qu'un faisceau d'observation et est enregistré au moyen d'un capteur (11), dans lequel, pendant la mesure de l'objet (2), l'optique de mise au point (6) est commandée de telle sorte que la distance de mise au point (8) du plan (7) net change progressivement par rapport à la caméra (1) entre plusieurs positions de balayage (12, 13, 14, 54) définies, dans lequel, pour chaque position de balayage (12, 13, 14, 54) au moyen du capteur (11), un premier enregistrement (35) et au moins un second enregistrement (36) sont effectués, dans lequel le capteur (11) est déplacé de manière oscillante, en un mouvement de va-et-vient, latéralement au trajet du faisceau du faisceau d'observation, dans lequel le premier enregistrement (35) est enregistré dans une première position de capteur (18) du capteur (11) et le second enregistrement (36) est enregistré dans une seconde position de capteur (19) du capteur (11), dans lequel le capteur (11) est décalé pendant le mouvement oscillant d'une distance (20) entre la première position de capteur (18) et la seconde position de capteur (19) le long d'un premier axe de capteur parallèle aux rangées des pixels de capteur ou le long d'un second axe de capteur parallèle aux colonnes des pixels de capteur, laquelle correspond à la largeur d'un pixel (33, 34) du capteur (11).

15. Procédé selon la revendication 14, dans lequel le motif de projection (30) est un motif en forme de damier et se compose d'éléments motifs (31, 32) carrés sombres et clairs ou de plusieurs bandes (41, 42) parallèles.
